(19) **Europäisches Patentamt**

**European Patent Office**

**Office européen des brevets**

(11) **EP 1 525 893 B1**

(12) **EUROPEAN PATENT SPECIFICATION**

(45) Date of publication and mention
of the grant of the patent:
**04.10.2006 Bulletin 2006/40**

(51) Int Cl.:
*A61M 15/00* (2006.01)   *B05B 7/00* (2006.01)

(21) Application number: **05075006.6**

(22) Date of filing: **26.10.1999**

(54) **Drug delivery apparatus**

Vorrichtung zur Abgabe von Medikamenten

Appareil d'administration de médicament

(84) Designated Contracting States:
**AT BE CH CY DE DK ES FI FR GB GR IE IT LI LU
MC NL PT SE**

(30) Priority: **26.10.1998 GB 9823434**

(43) Date of publication of application:
**27.04.2005 Bulletin 2005/17**

(62) Document number(s) of the earlier application(s) in
accordance with Art. 76 EPC:
**99950992.0 / 1 124 602**

(73) Proprietor: **Respironics (UK) Limited
Bognor Regis,
West Sussex PO22 9SL (GB)**

(72) Inventors:
• **Denyer, Jonathan Stanley Harold
Chichester
West Sussex PO19 3PW (GB)**

• **Dyche, Anthony
Hayling Island
Hampshire PO11 0ND (GB)**
• **Prince, Richard Ivan
Hedge End
Southampton SO30 0FD (GB)**

(74) Representative: **Wright, Howard Hugh Burnby et al
Withers & Rogers LLP
Goldings House,
2 Hays Lane
London SE1 2HW (GB)**

(56) References cited:
EP-A- 0 667 168     WO-A-92/11054
WO-A-97/48431     DE-A- 3 636 669
GB-A- 2 077 444     GB-A- 2 294 402
US-A- 3 741 208

**Description**

This invention relates to drug delivery apparatus, particularly, but not exclusively, nebulisers and dosimetric spacers.

Many different types of nebulisers are known for delivering medication directly into the lungs of a patient, usually for treatment of respiratory diseases. Nebulisers normally deliver medication in the form of droplets or a dry powder. In most nebulisers, atomisation of the medicament into a stream of air occurs continuously, regardless of whether the patient is inspiring or expiring. However, the effect of continuous atomisation is that a significant proportion of the medication is lost during expiration.

Commonly known nebulisers are either pneumatically operated from a compressed air source connected to the nebuliser which atomises the liquid, or are ultrasonic nebulisers which use a piezo-electric crystal to atomise the liquid. More recently, a mesh-type nebuliser has been developed in which the medication is forced through a fine mesh in order to create droplets of the medication. A further type of nebuliser, or inhaler, is one which uses a piezo-electric vibrator together with an electro-static charge plate to fluidise and disperse a dry powder aerosol into an airstream. Such a nebuliser is disclosed in US 5694920.

The optimum diameter of medication particles or droplets is about 1-5 microns. If the particles or droplets are bigger than this, they are likely to be impacted in the airway before they reach the lungs, but if they are smaller than one micron, they tend to be carried out of the lungs again on exhalation without sedimenting in the lungs.

Nebulisers and inhalers disperse the small particles of medication into an air stream, or stream of other gas, leading to a patient. References to the air which carries the medication entrained in it includes other gases suitable for carrying the medication.

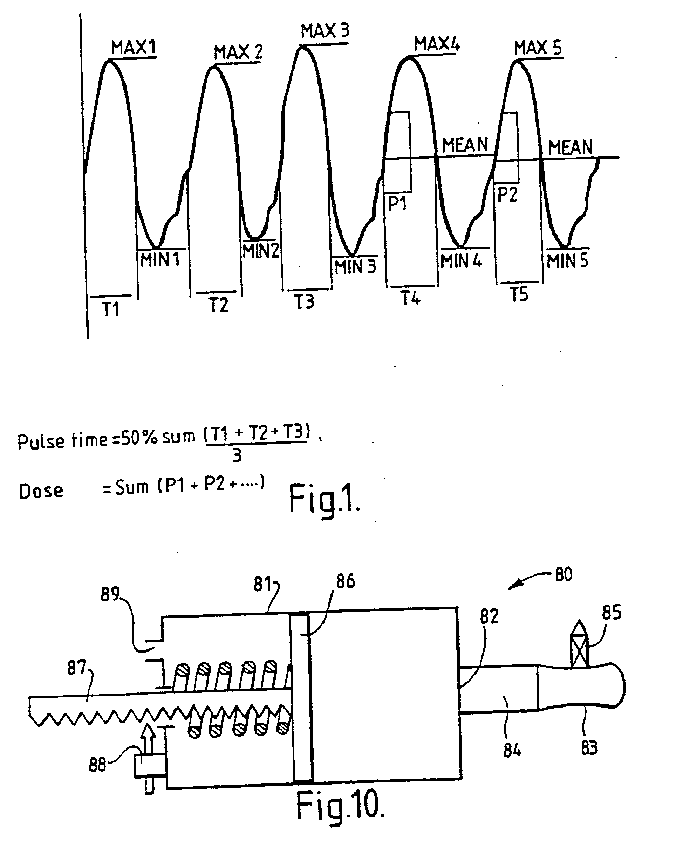

One known nebuliser analyses the pressure changes within the device during the first three breaths to determine an average shape of the breathing pattern. A timed pulse of atomisation is commenced when starting subsequent inspirations such that atomisation occurs for the first 50% of the inspiration. This is illustrated in Figure I where the breathing pattern and pulse are superimposed. This is effective in reducing the loss of medication during exhalation to about 3%. Figure 1 shows the breaths in a graph of flow rate against time. When the treatment is commenced, a patient breathes in and out three times through the nebuliser before treatment commences. The first three breaths are measured so that the timed pulse of atomisation occurs for 50% of the average time of inhalation. The duration of inhalation is indicated as T1, T2 and T3. These timed periods are averaged, and divided by two in order to determine the pulse length for the next fourth breath where treatment starts. For each subsequent breath, the duration of the pulse of atomisation is determined by summing the time period of inhalation of the previous three breaths, dividing by three to obtain an average and dividing by two. The dose administered to the patient is directly proportional to the duration of the pulse of atomisation, and so the period of atomisation is summed, and the atomiser is switched off, or indicates that the patient should stop once the dose administered to the patient reaches the amount of medication prescribed for that treatment.

Other nebulisers are known in which the timed pulse of atomisation is fixed to be other than 50% of the duration of inspiration. However, in these other nebulisers, the pulse length must be set for each patient by the clinician. Many of the nebulisers are, therefore, suitable only for use in a controlled environment, such as a hospital. The setting of the pulse length for each patient means that most nebulisers are not suitable for a patient to use at home.

Reference is made to our co-pending International Patent Publication No. WO 97/48431. Figures 2 and 3 of this application show the nebuliser which is disclosed in the above co-pending Patent application. Referring to Figure 2, a mouthpiece 1 is shown through which a patient inhales in the direction of arrow 2. Below the mouthpiece 1 is a removable atomising section 3 which, in turn, rests on a base 4.

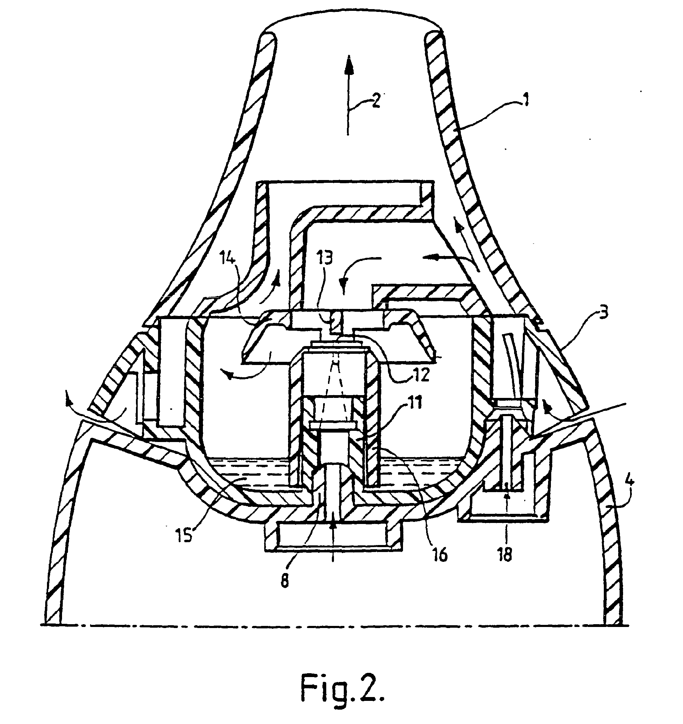

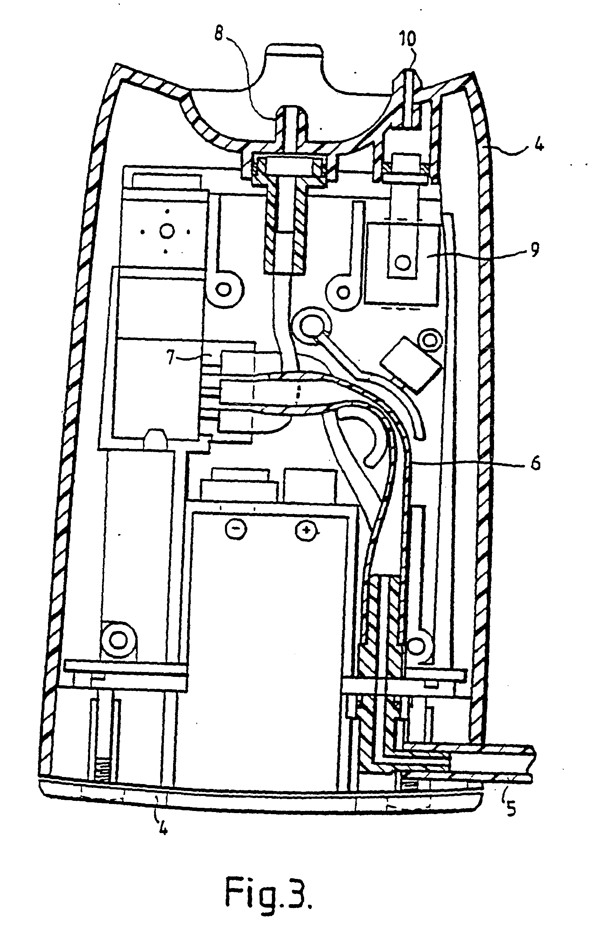

The base 4 is shown in more detail in Figure 3. Referring to Figure 3, the base 4 includes an inlet 5 through which air is supplied under pressure from a compressor (not shown). The pressurized air is led via a tube 6 to a manifold 7 which controls the flow of pressurized air to an air outlet 8 which directs air into the atomising section 3 shown in Figure 2. The base 4 also includes a pressure sensor 9 which detects the pressure within the atomising section 3 via a port 10.

Referring again to Figure 2, air under pressure passes through the air outlet 8 of the base 4 and is conducted through a tubular post 11 to an atomiser nozzle 12 out of which the air issues under pressure. A deflector 13 is located in the path of the pressurised air issuing from the nozzle 12 so that the pressurized air is deflected laterally so as to pass beneath a baffle 14. The passage of the pressurized air across the top of the tubular post 11 causes medication 15 to be drawn up between the outer surface of the tubular post 11 and the inner surface of a sleeve 16 which surrounds the tubular post 11. The medication 15 is atomised in the stream of air, and carried away in the stream of air below the rim of the baffle 14 and up through the mouthpiece 1 to a patient.

The pressure sensor 9 in the base 4 monitors the breathing pattern of a patient, and on the basis of the breathing pattern, the manifold 7 is controlled to supply pressurized air to the atomising section 3 only during the first 50% of an inhalation phase.

Whilst a particular type of nebuliser is described above, the present application is suitable for application to any type of nebuliser.

The invention also relates to other drug delivery apparatus, such as spacers in which a dose of a drug in droplet or powder form is released into a spacer chamber or holding chamber from which the patient inhales. These are most appropriate for elderly patients or children who have difficulty in using a multi-dose inhaler or dry powder inhaler, for example, because they have trouble coordinating the release of the drug with the beginning of inhalation, or because their inhalation flow rates are too small. For example, spacers are disclosed in International patent publication number WO 96/13294.

According to a first aspect of the present invention, a drug delivery apparatus comprises means for predicting the tidal volume including means arranged to measure a patient's peak flow, a timer arranged to measure the duration of inspiration, and a tidal volume predictor arranged to calculate the tidal volume on the basis of the peak flow measured by the peak flow measuring means, and the duration of inspiration measured by the timer.

According to a second aspect of the invention, a non-therapeutic method of predicting the tidal volume of a patient with the above described drug delivery apparatus comprises :

    (i) measuring the patient's peak flow;
    (ii) measuring the duration of inspiration of a patient;
    (iii) calculating the tidal volume on the basis of the measured peak flow, and the measured duration of inspiration of the patient,

whereby no drug is delivered to the patient.

Measuring the patient's respiratory volume (tidal volume) has previously involved continually monitoring the patient's inspiratory flow, typically every ten milliseconds. The flow rate is integrated over the duration of inspiration to determine the inspiratory volume. However, the invention determines the tidal volume of a patient much more simply. This invention reduces the amount of data processing which is required, thereby reducing the cost of the overall nebuliser. The peak flow is much simpler to measure, and can be used more simply in a calculation to determine the tidal volume.

Some or all of the values used in the calculations are mean values derived from a number of earlier measurements of each breathing pattern of the patient. For example, the patient will start inspiration through the apparatus, and the medication will not be delivered during the first three breaths. The first three breaths are analysed by recording the duration of inspiration, and the peak flows during inhalation as are required to determine the duration of a pulse of atomisation. Delivery of the medication takes place on the fourth and subsequent breaths, in each case the values in the calculations are derived from a number of earlier measurements of the inspiration phase of a patient, in this case the previous three inspiratory phases.

Preferably, where the apparatus is a nebuliser, the atomisation is caused by a stream of gas under pressure passing through the nebuliser and sourced from a gas supply means. This gas is normally air, and the source is preferably a compressor operating together with an accumulator. During atomisation, gas from the accumulator is used to atomise the medication, and the compressor generates air under pressure to fill the accumulator. If a patient's inspiration is very long, the accumulator may be caused to be emptied, thereby disrupting atomisation. The atomiser, therefore, preferably includes a means for limiting the duration of the pulse so as to maintain the accumulator in a state where it is always under some pressure. In addition, the accumulator may include a valve which, when the accumulator is full, vents gas to atmosphere thereby preventing it from becoming dangerously full. It is often preferable to maintain the compressor in operation all the time and to vent excess air to atmosphere rather than to switch the compressor on or off.

In this document, the upper airways of a patient are the mouth and trachea, and where a nebuliser is used, preferably include the volume of the nebuliser chamber.

The determination of the length of pulse enables the proportion of the inhalation time during which atomisation occurs to be extended above 50% towards 100%. This will result in the patient receiving their treatment in a shorter time, since it will take fewer breaths to deliver the required dose of medication. However, there is no point in continuing delivering the medication into air which is inhaled by the patient at the end of his or her inspiratory phase (the 'end volume'), since it will remain in the upper airways. The medication which does not go beyond the upper airways will be wasted when the patient exhales.

Thus, it is advantageous for a pulse of medication-laden air to be generated which is longer than 50% but which stops before the end volume of inspiration begins. This has the advantage that a patient's adherence to the treatment regime will be much improved if the length of treatment is reduced.

In addition, automatic optimisation of the pulse length can be achieved, without it needing to be set by a clinician. This means that the pulse length will automatically be adapted to each patient on the basis of the patient's breathing pattern at the time the medication is being administered. Thus, a nebuliser or other drug delivery apparatus may be used by the patient outside of the controlled environment of a hospital, and may be used at home. In addition, it is possible for the apparatus to indicate when a dose has been administered without the patient needing to count the number of breaths which he or she has taken.

Embodiments of the present invention are described below by way of example, and with reference to the drawings in which:

Figure 1 is a graph showing the inhalation pattern of a patient over time, and indicating when the pulse of atomisation occurs in the first 50% of inspiration, as occurs in one known nebuliser;

Figures 2 and 3 show a known nebuliser which generates pulses of atomisation during the first 50% of inspiration;

Figure 4 is a flow diagram showing how the pulse of atomisation during inspiration is determined;

Figure 5 is a graph showing the predicted tidal volume against measured tidal volume;

Figure 6 is a flow diagram showing the limitation of the pulse length depending on the supply of pressurised gas;

Figure 7 shows the nebuliser together with a source of pressurised gas;

Figure 8 shows an air accumulator within the air supply;

Figure 9 is a schematic drawing showing the way in which the nebuliser is controlled; and

Figure 10 is a schematic drawing of a dosimetric spacer according to the present invention.

This invention applies, amongst other things, to nebulisers of the type which generate pulses of atomisation, as in the prior art nebuliser described above. The invention is not, however, limited to the exact nebuliser described above, but may be applied to other nebulisers. For convenience, the description below of the present invention will refer to components of the prior art device shown in Figures 2 and 3, and because many of the components, for example, the manifold, may be used in the present invention. The nebuliser may be one of a jet nebuliser, ultrasonic nebuliser or a pressure mesh nebuliser.

Jet nebulisers are of two kinds, these being air-jet nebulisers and liquid-jet nebulisers. An example of an air-jet nebuliser, which uses a source of compressed air to nebulise a liquid, is disclosed in EP 0627266 (Medic-Aid Limited). An example of a liquid-jet nebuliser, which drives a liquid through one or more nozzle outlets to produce a spray of fine droplets is disclosed in WO 94/07607 (Boehringer Ingelheim International GmbH et al).

Ultrasonic nebulisers which nebulise a liquid using ultrasonic waves usually developed with an oscillating piezo-electric element, take many forms, these including nebulisers where liquid is in direct contact with the piezo-electric element, where there is an amplifying interface, typically an enclosed fluid, between the piezo-electric element and the liquid, and where the piezo-electric element vibrates a mesh from which an aerosol is generated. Examples of ultrasonic nebulisers are disclosed in US 4533082 (Maehara et al) and US 5261601 (Ross et al). The nebulisers described in those documents include a housing that has a reservoir which holds a quantity of liquid to be dispensed, which housing has a perforated membrane in contact with the reservoir and an ultrasonic vibrator connected to the housing to vibrate the perforated membrane. Another example of an ultrasonic nebuliser is described in WO 97/29851 (Fluid Propulsion Technologies, Inc). An example of a pressure mesh nebuliser, which may or may not include a piezo-electric element, is disclosed in WO 96/13292 (Aradigm Corporation).

Extending the proportion of the inhalation of the patient in which atomisation takes place above 50% results in the patient receiving their treatment faster since it will take fewer breaths to deliver the required volume of medication. However, to avoid wastage of the medication which is atomised in the end volume of patient's inspiratory volume, the pulse of atomisation must be stopped before the end volume is reached. The end volume is the volume of air inhaled by a patient at the end of the inspiratory volume which remains in the upper airways (the mouth and trachea) and does not enter into the lower parts of the lungs. Medication which is atomised into the end volume is wasted when the patient exhales, together with any air atomised medication left in the nebuliser, since it does not reach the lungs.

The end volume is the volume of the patient's upper airway, and is proportional to the size of the patient. Clearly, the end volume will vary as a percentage of the inspiratory tidal volume since the tidal volume changes significantly depending on the type and extent of the respiratory disease suffered by the patient. The optimum duration of atomisation pulse would, therefore, be from the start of inhalation up to the point during inspiration when the volume remaining to be inspired equals the end volume. Atomisation would then be stopped and the remaining end volume would clear the atomised medication from the device and the upper airways of the patient and into the lungs. Thus, the percentage of inspiration in which atomised medication is delivered is maximised, thereby minimising treatment time and still avoiding wastage of medication. The length of the atomisation pulse is dependent upon the patient's inspiratory tidal volume. The nebuliser must therefore measure the patient's tidal volume, preferably on a breath by breath basis so as to calculate, for example from the previous three breaths, an average inhalation volume for the next breath. Thus, the atomisation pulse time will be calculated as follows:

$$\text{Pulse time} = \text{mean inspiratory time} \times \frac{(\text{mean tidal volume} - \text{end volume})}{\text{mean tidal volume}}$$

Timing means are included in the nebuliser connected to the pressure sensor 9 (shown in Figure 3) in order to measure the duration of inspiration. Storage means are also included in the nebuliser in which an estimate of the end volume of a particular patient is stored. Since this figure is a constant value for a particular patient, this can be entered at the beginning of a course of treatment, and is estimated on the basis of the size of the patient. The nebuliser includes a means for measuring the tidal volume of a patient. According to one form of the invention, the patient's inspiratory flow is monitored continuously, typically every ten milliseconds, and this is integrated over the inspiratory duration. Another, simpler, way of measuring the tidal volume of a patient is described later in this specification.

The nebuliser also includes means for calculating the atomisation pulse time on the basis of the duration of inspiration, the tidal volume and the end volume. The calculation means carries out the calculation outlined above.

In view of the fact that the nebuliser adapts to the breathing pattern of a patient, when the patient starts breathing, no atomisation takes place during the first three breaths. Those first three breaths are used to analyse the breathing pattern of the patient. The flow rate of the first three breaths are measured, and from this, the duration of the inhalation phase of the first three breaths are calculated, and an average found. The average duration of inhalation is then used in the calculation to determine the pulse length of atomisation during the fourth breath. In addition, as the patient continues to breathe in and out, the previous three breathing patterns are measured and used to calculate the next pulse duration. Thus, if a patient's breathing pattern improves during treatment, the nebuliser will adapt to this change in order to optimise the dose administered during each breath.

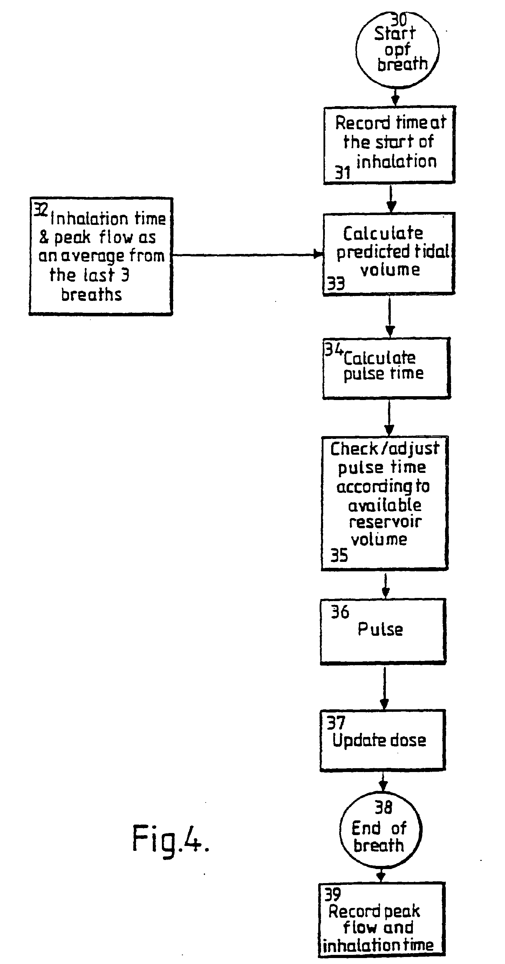

Referring now to Figure 4, the steps taken by the nebuliser, and by the patient are described. The first operation, box 30 represents the patient starting to inhale. The timing means records the time at which inhalation starts as shown in box 31, and during inhalation, a calculation is performed to predict the tidal volume of the patient as shown in box 33. This step will be described in more detail later in a specification, but it will be noted that the calculation requires data to be included in the calculation which is the inhalation time and peak flow as an average from the last three breaths, as shown in box 32. The pulse time is then calculated by the calculation means as shown in box 34, and the pulse time is adjusted, as shown in box 35 in the event that the pulse length would exhaust an accumulator from which is pressurised air is supplied to the nebuliser. This step, shown in box 35 is also described in more detail later in this specification. The pulse of atomisation occurs during inhalation, and after it has stopped, a calculation is carried out to determine what dose has been atomised. At the end of the breath as shown in box 38, details of the peak flow of the patient inhalation, and the duration of inhalation are recorded so that calculations determining pulse length may be made for subsequent breaths. This is shown in box 39.

Reference is made above to the simpler prediction of tidal volume. As will be appreciated, measuring tidal volume by integrating measured flow rate over the time of inspiration requires considerable processing power and is relatively expensive. A simpler method of determining the tidal volume is proposed which requires much simpler calculations and much simpler measurements to be made for use in such a calculation. To carry out the measurement, the nebuliser includes a peak flow detector for detecting the peak flow rate of inspiration.

The calculated, or predicted tidal volume is derived from the peak flow measured by the peak flow detector, and the duration of inspiration measured by the timer. The tidal volume calculation means carries out the following calculation:

$$\text{Predicted tidal volume} = C \times \text{Mean Peak Flow} \times \frac{\text{Inspiratory Time}}{60}$$

C is a constant and it is found that C = 0.7

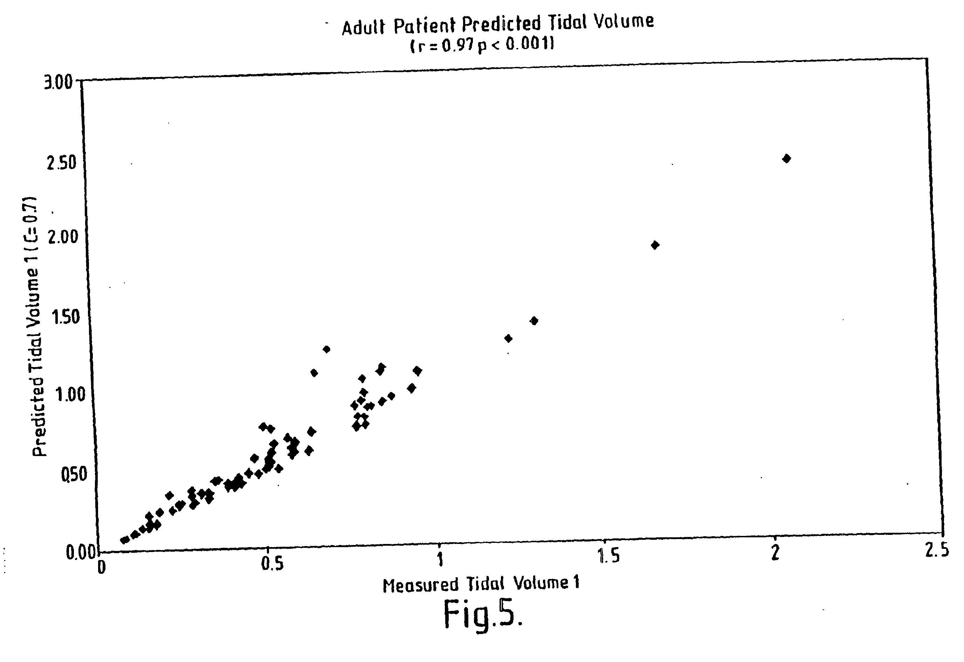

Figure 5 is a graph of the predicted tidal volume against measured tidal volume. Each point on the graph represents a patient whose tidal volume has been measured by a complex tidal volume calculation by integration of the patient's inspiratory flow over the duration of inhalation, and the predicted tidal volume according to the new, simpler method of calculation. It will be seen that the predicted tidal volumes are extremely accurate, and so the predicted tidal volume may be included in the calculation of atomisation pulse time.

The use of a low flow rate compressor together with an accumulator to supply compressed air to the nebuliser is disclosed in our earlier Patent application published as WO 97/48431, which is referred to above. In the past, the size of the compressor and accumulator are selected so that the maximum pulse that can be delivered by the device (currently 50% of inspiratory time) does not exceed the accumulator volume for any given pulse or the mean output of the compressor. Now that the pulse time is variable, it is preferable to calculate the maximum pulse time available from the air supply system. For patients who have a slightly higher inspiratory demand, the pulse time of atomisation will be reduced so that the supply capability of the air supply system is not exceeded. The calculations are carried out on a breath by breath basis, assuming that the accumulator is filled at constant flow rate from the compressor. The volume of air added to the accumulator from the end of previous pulse to the start of the next pulse is calculated and then added to the volume remaining at the end of the previous pulse.

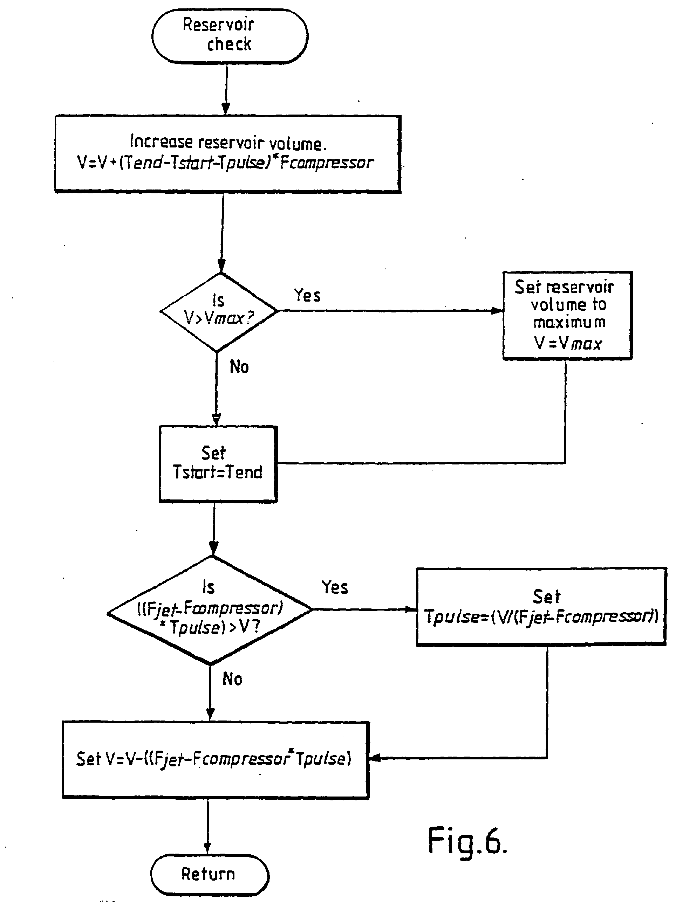

Figure 6 is a flow diagram showing the calculations carried out in ensuring that the volume of air used does not exceed the volume of the accumulator. If the air in the accumulator is calculated to be above the maximum volume of the accumulator, the volume is set to be at its maximum $V = V_{max}$. This is because there is an automatic vent valve which limits the volume of air stored in the accumulator. The maximum pulse time can then be calculated on the basis of the rate of flow of air out of the accumulator which is the flow to the atomiser jet, minus the flow rate to the compressor. If this exceeds the volume available in the accumulator, then the pulse time is limited to the current accumulator volume. The volume of accumulator at the end of the pulse is then calculated to be used at the beginning of the next calculation occurring at the beginning of the next inhalation of the patient. Thus, the maximum pulse time for individual breaths is calculated without exceeding the capacity of the air supply system. The compressor has a constant output flow rate, typically 1.5 litres per minute and the nebuliser jet has a flow rate of 6 litres per minute during pulsing. The accumulator has a volume of approximately 150 millilitres at NTP.

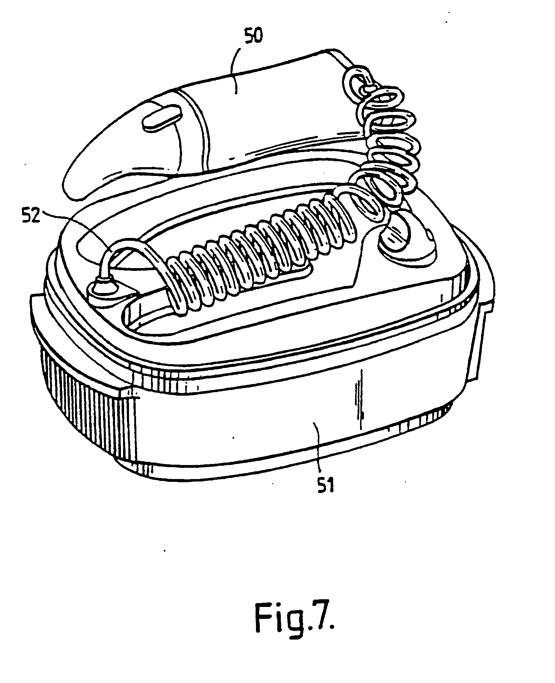

Figure 7 shows the nebuliser 50 connected to the air supply 51 by a flexible tube 52.

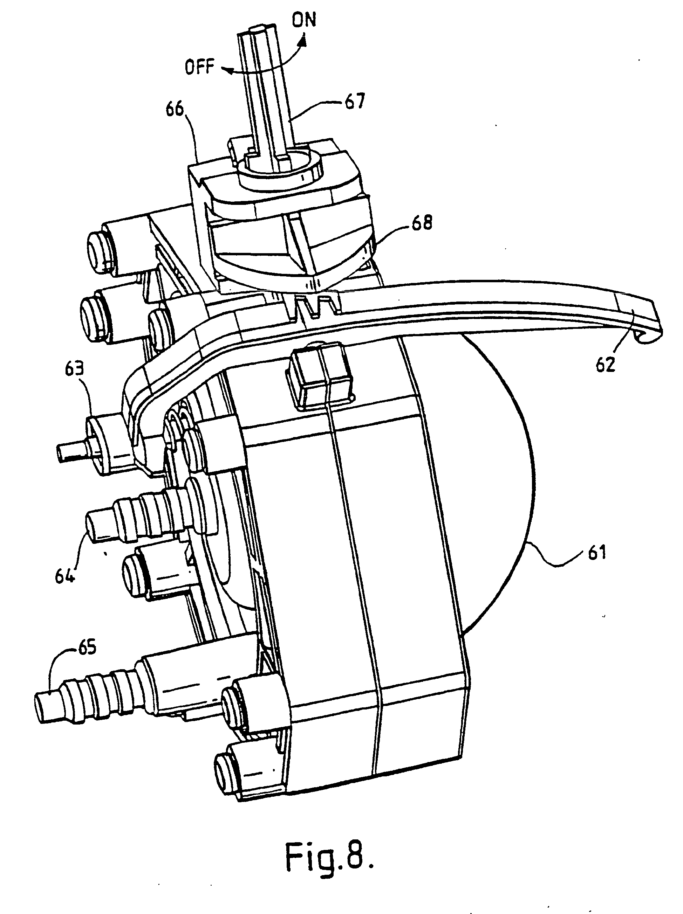

Referring to Figure 8, the accumulator is shown which has a vent 63, thereby limiting the maximum expansion of the accumulator. As each pulse is delivered to the nebuliser, the diameter of the accumulator is reduced, and the vent 63 is closed.

The compressor may be mains powered or battery powered. The pump, especially a mains powered pump, operates continually during use, and operates to inflate the accumulator. When the pressure in the accumulator reaches the required level, a pressure switch in the hand-held part of the nebuliser is activated as is described in an earlier Patent application referred above. That switches the nebuliser ON. Once the treatment has been completed, the compressor is switched OFF. The accumulator deflates and the pressure switch in the hand-held part of the nebuliser deactivates the unit.

Referring to Figure 8, the pump supplies air to the accumulator via a port 64. Inflation of the diaphragm 61 of the accumulator is controlled by an assembly including an arm 62 which is connected to a vent valve 63. When the diaphragm 61 of the accumulator reaches the maximum desired extension, it contacts the arm 62 to open the vent valve 63. This releases to atmosphere the flow of air from the compressor, and maintains the accumulator at a fixed extension. During use, air is removed from the accumulator via port 65 and the diaphragm 61 shrinks and loses contact with the vent arm 62 which closes the valve 63 allowing the compressor to recharge the accumulator until the vent arm 62 again operates the vent valve 63.

It is also advantageous to vent the accumulator to atmosphere when the compressor is switched off, and this is achieved by mounting the main power switch 66 on top of the accumulator with a rotary knob 67. The bottom of the knob 67 includes a cam 68 which contacts the vent arm 62 to open the vent 63 thereby releasing pressure from the accumulator. Simultaneously, the compressor is switched off. When the compressor is switched back on again, the cam 68 is disengaged from the vent arm 62, thereby closing the vent valve 63.

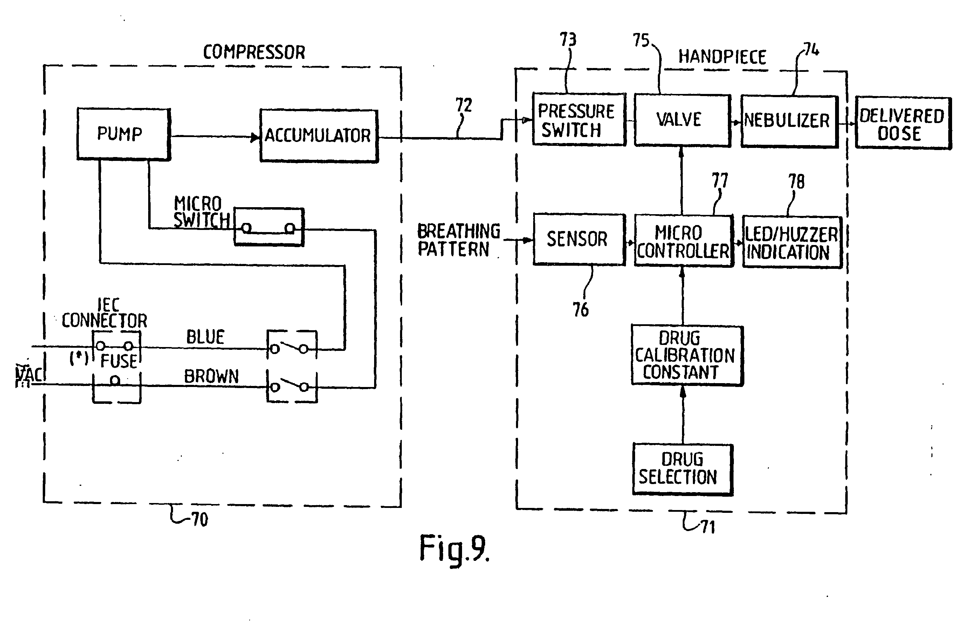

Figure 9 illustrates a simplified form of the way in which all of the components of the nebuliser are connected together. The compressor and accumulator 70 are shown as being separate from the hand-held part of the nebuliser 71, but connected by a tube 72 carrying the pressurised air into the nebuliser 71. In the compressor and accumulator part 70, the pump is shown to supply the accumulator 70 with compressed air. In the nebuliser part 71, the nebuliser is switched on at the pressure switch 73 by the presence of pressurised air in tube 72. The nebulising part 74 of the nebuliser is controlled by a valve or manifold 75 which controls the pulses of pressurised air. The breathing pattern of a patient is detected by a sensor 76 which delivers information regarding the breathing pattern to the micro-controller 77 which, in turn, controls the manifold 75. Once a dose of medication has been delivered, indication means, such as a LED or buzzer 78 is activated by the micro-controller to indicate to the patient that treatment is complete.

A further embodiment of the invention is shown in Figure 10 which is a dosimetric spacer 80 including a holding chamber 81 having a port 82 towards one end thereof to which is connected a mouthpiece 83. An air pressure sensor 84 is located between the mouthpiece 83 and holding chamber 81. This sensor 84 measures the pressure within the mouthpiece, from which the rate of flow of air inspired and exhaled by the patient can be measured air. The mouthpiece 83 also includes a vent 85 which allows a patient to exhale through the mouthpiece 83 without filling the holding chamber 81. More is disclosed on the vent below.

Within the holding chamber, a piston 86 is disclosed to move longitudinally to vary the volume of air available in the holding chamber 81 to a patient during inspiration. The piston includes a toothed connecting rod 87 which extends through the end of the holding chamber 81 such that the teeth may be engaged by the finger of solenoid 88. An air inlet 89 is located at the left hand end of the holding chamber in order to allow air to enter or leave the space behind the piston as the piston moves to the right or left.

In use, the piston 86 is pulled back so as to fill the holding chamber 81 with air. The air within the holding chamber 81 is then loaded with medication, either in the form of liquid droplets, or in the form of a cloud of powder. This is delivered into the holding chamber 81 through the port 82, and normally requires the removal of the mouthpiece 83 to do so. A mouthpiece 83 then can be replaced, and a patient breathes in and out through the mouthpiece 83. During inspiration, a patient inspires the medication-laden air from the holding chamber 81, and during exhalation, the exhaled air is vented to atmosphere by the vent 85. During exhalation, the solenoid 88 locks the connecting rod 87 of the piston 86 so that it will not move and so that the holding chamber will not fill with exhaled air. However, in accordance with the invention, the piston 86 is only free to move during a proportion of the inhalation phase, and it will be locked stationary by the solenoid 88 during the inspiration by the patient of the end volume. Once the piston locks, the vent 85 is arranged such that the drop in pressure in the mouthpiece 83 caused by the locking of the piston 86 opens the vent 85 such that ambient air may be drawn into the mouthpiece. Of course, a separate vent might be included in the mouthpiece to carry out this function as appropriate.

The calculation of the pulse length during which the piston 86 is free to move to allow the dispensing of medication to the patient is determined in the same way as is described above in relation to the nebuliser. The inspiration of the patient during the previous three breaths is monitored by the sensor 84 such that the same calculations can be made as described above. On the subsequent breath, the sensor detects the commencement of a breath, and after the duration of the pulse, the piston is locked.

Such an arrangement reduces the wastage of medication which is present in the end volume of the air normally breathed in by the patient.

This invention is applicable to other types of medical inhalers. For example, as described in the introduction to this specification, a dry powder inhaler is disclosed in US 5694920 which uses a piezo-electric vibrator and an electrostatic charge plate to fluidise and disperse a dry powder into the air stream of the patient. The electrostatic charge plate can be operated in response to the patient's breathing pattern so as to produce pulses of the powder medication into the air stream leading to the patient. The length of the pulses can be determined in exactly the same way as in the embodiments previously described such that the dry powder is not dispersed into the end volume of the air stream leading to the patient.

## Claims

1. Drug delivery apparatus comprising means for predicting a patient's tidal volume **characterised by** means arranged to measure a patient's peak flow, a timer arranged to measure the duration of inspiration, and a tidal volume predictor arranged to calculate the tidal volume on the basis of the peak flow measured by the peak flow measuring means, and the duration of inspiration measured by the timer.

2. Apparatus according to claim 1, wherein some or all of the values used in the calculations are mean values derived from a number of earlier measurements of the breathing pattern of the patient.

3. Apparatus according to claim 1 or 2, wherein the apparatus is a nebuliser.

4. A nebuliser (50) according to claim 3, further comprising means for determining the duration of a pulse of atomisation during inspiration, the determination means including the said means for predicting the tidal volume, means for storing an estimate of the volume of a patient's upper airway, and means for calculating the duration of the pulse on the basis of the tidal volume determined by the means for predicting the tidal volume , the duration of inspiration measured by the timing means, and the stored estimated volume of a patient's upper airway from the storage means.

5. A nebuliser (50) according to claim 3, further comprising:

   means for atomising a medication;
   means for monitoring a patient's breathing pattern; and
   means for controlling the atomising means to atomise the medication in pulses,
   wherein the length of the pulses, and their proportion of the inspiratory phase of the breathing pattern are varied by the controlling means depending on the breathing pattern monitored by the monitoring means.

**6.** Apparatus according to claim 1 or 2, wherein the apparatus is a spacer.

**7.** A method of predicting the tidal volume (33) of a patient with an apparatus according to any of claims 1-6, comprising :

(i) measuring a patient's peak flow;
(ii) measuring the duration of inspiration of a patient;
(iii) calculating the tidal volume on the basis of the measured peak flow, and the measured duration of inspiration of the patient; whereby no drug is delivered to the patient.

**8.** A method according to claim 7, wherein calculations are carried out on mean values derived from a number of earlier measurements of the breathing pattern of the patient.

**Patentansprüche**

**1.** Vorrichtung zur Abgabe von Medikamenten, umfassend Mittel zur Vorhersage des Atemvolumens eines Patienten, **gekennzeichnet durch** eine Einrichtung zur Messung der Maximalatemströmung eines Patienten, einen Zeitnehmer zur Messung der Dauer der Einatmungsphase und einen Atemvolumen-Vorausberechner zur Berechnung des Atemvolumens auf der Grundlage der **durch** die Maximalatemströmung-Meßeinrichtung gemessenen Maximalatemströmung und der vom Zeitnehmer gemessenen Dauer der Einatmungsphase.

**2.** Vorrichtung gemäß Anspruch 1, bei welcher einige oder alle der in den Berechnungen verwendeten Werte Mittelwerte sind, die aus einer Anzahl vorhergehender Messungen des Atemmusters des Patienten gewonnen werden.

**3.** Vorrichtung gemäß Anspruch 1 oder 2, welche Vorrichtung ein Zerstäuber ist.

**4.** Zerstäuber (50) gemäß Anspruch 3, ferner umfassend eine Einrichtung zur Bestimmung der Dauer eines Zerstäubungsstoßes während der Einatmungsphase, welche Bestimmungseinrichtung die genannte Einrichtung zur Vorhersage des Atemvolumens umfaßt, sowie Mittel zu Speicherung eines Schätzwerts des Volumens eines oberen Luftwegs des Patienten und eine Einrichtung zur Berechnung der Dauer des Stoßes auf der Grundlage des Atemvolumens, das durch die Einrichtung zur Vorhersage des Atemvolumens bestimmt wird, sowie der von der Zeitnehmereinrichtung gemessenen Dauer der Einatmungsphase sowie des von der Speichereinrichtung gespeicherten geschätzten Volumens eines oberen Luftwegs des Patienten.

**5.** Zerstäuber (50) gemäß Anspruch 3, ferner umfassend:

eine Einrichtung zur Zerstäubung eines Medikaments;
eine Einrichtung zur Überwachung eines Atemmusters
eines Patienten; und
eine Einrichtung zur Steuerung der Zerstäubungseinrichtung zur Zerstäubung des Medikaments in Stößen,

wobei die Länge der Stöße und ihr Anteil an der Einatmungsphase des Atemmusters durch die Steuereinrichtung in Abhängigkeit von dem durch die Überwachungseinrichtung überwachten Atemmuster variiert werden.

**6.** Vorrichtung gemäß Anspruch 1 oder 2, welche Vorrichtung ein Abstandstück ist.

**7.** Verfahren zur Vorhersage des Atemvolumens (33) eines Patienten mittels einer Vorrichtung gemäß einem der vorhergehenden Ansprüche 1 bis 6, umfassend die folgenden Schritte:

(i) Messung einer Maximalatemströmung eines Patienten;
(ii) Messung der Dauer der Einatmungsphase eines Patienten;
(iii) Berechnung des Atemvolumens auf der Grundlage der gemessenen Maximalatemströmung und der gemessenen Dauer der Einatmungsphase des Patienten, wobei an den Patienten kein Medikament verabreicht wird.

**8.** Verfahren gemäß Anspruch 7, bei welchem Berechnungen anhand von Mittelwerten durchgeführt werden, die aus einer Anzahl vorhergehender Messungen des Atemmusters des Patienten gewonnen werden.

## Revendications

1. Appareil d'administration de médicament comprenant des moyens de prédiction du volume respiratoire courant d'un patient, **caractérisé par** des moyens agencés pour mesurer un débit de pointe d'un patient, un minuteur agencé pour mesurer la durée d'inspiration et un dispositif de prévision de volume respiratoire courant agencé pour calculer le volume respiratoire courant sur la base du débit de pointe mesuré par les moyens de mesure de débit de pointe et de la durée d'inspiration mesurée par le minuteur.

2. Appareil selon la revendication 1, dans lequel certaines valeurs, ou l'ensemble des valeurs, utilisées dans les calculs sont des valeurs moyennes dérivées d'une série de mesures antérieures du cycle respiratoire du patient.

3. Appareil selon la revendication 1 ou 2, dans lequel l'appareil est un nébuliseur.

4. Nébuliseur (50) selon la revendication 3, comprenant en outre des moyens pour déterminer la durée d'une impulsion d'atomisation pendant l'inspiration, les moyens de détermination comprenant lesdits moyens pour prévoir le volume respiratoire courant, des moyens pour mémoriser une estimation du volume de la voie respiratoire supérieure d'un patient et des moyens pour calculer la durée de l'impulsion sur la base du volume respiratoire courant déterminé par les moyens de prévision du volume respiratoire, de la durée d'inspiration mesurée par les moyens de mesure de temps et du volume estimé de la voie respiratoire supérieure d'un patient mémorisé par les moyens de mémorisation.

5. Nébuliseur (50) selon la revendication 3, comprenant en outre :

   des moyens d'atomisation d'un médicament ;
   des moyens de contrôle du cycle respiratoire d'un patient ; et
   des moyens de commande des moyens d'atomisation pour atomiser le médicament par impulsions, dans lequel la longueur des impulsions et leur proportion dans la phase inspiratoire du diagramme de respiration sont ajustables à l'aide des moyens de commande en fonction du rythme respiratoire contrôlé par les moyens de contrôle.

6. Appareil selon la revendication 1 ou 2, dans lequel l'appareil est un dispositif d'espacement.

7. Procédé de prédiction du volume respiratoire (33) d'un patient à l'aide d'un appareil selon l'une quelconque des revendications 1 à 6, ledit procédé comprenant les étapes consistant à :

   (i) mesurer un débit de pointe d'un patient ;
   (ii) mesurer la durée d'inspiration d'un patient ;
   (iii) calculer le volume respiratoire courant sur la base du débit de pointe mesuré et de la durée d'inspiration mesurée du patient ; moyennant quoi aucun médicament n'est administré au patient'.

8. Procédé selon la revendication 7, dans lequel les calculs sont réalisés sur des valeurs moyennes dérivées d'une série de mesures antérieures du cycle respiratoire du patient.

$$\text{Pulse time} = 50\% \text{ sum } \frac{(T1 + T2 + T3)}{3}$$

$$\text{Dose} = \text{Sum } (P1 + P2 + \ldots)$$

Fig.1.

Fig.10.

Fig.2.

Fig.3.

Fig.4.

## Adult Patient Predicted Tidal Volume
### (r = 0.97 p < 0.001)

Fig.5.

EP 1 525 893 B1

Fig.6.

Fig.7.

Fig.8.

COMPRESSOR

PUMP → ACCUMULATOR

MICRO SWITCH

IEC CONNECTOR

(*) FUSE

VAC

BLUE

BROWN

72

73 75 HANDPIECE 74

PRESSURE SWITCH → VALVE → NEBULIZER → DELIVERED DOSE

BREATHING PATTERN → SENSOR → MICRO CONTROLLER → LED/HUZZER INDICATION

77 78

76

DRUG CALIBRATION CONSTANT

DRUG SELECTION

70

71

Fig.9.

EP 1 525 893 B1

18